# EUROPEAN PATENT SPECIFICATION

(11) **EP 2 881 113 B1**
(45) Date of publication and mention of the grant of the patent: **10.04.2019**
(21) Application number: 13825879.3
(22) Date of filing: 29.07.2013
(51) Int. Cl.: A61K 31/366, A61K 36/53, A61P 31/02, A61P 31/04, A61P 31/10, A61K 8/49, A23L 3/3499, A61Q 17/00, A01N 49/00

(54) **NEW APPLICATION OF POGOSTONE**
NEUE VERWENDUNG VON POGOSTON
NOUVELLE APPLICATION DE LA POGOSTONE

(30) Priority: 30.07.2012 CN 201210268204
(43) Date of publication of application: 10.06.2015
(73) Proprietor: Chengdu Taihe Health Technology Group Inc., Ltd., Cheng-du Sichuan 610041 (CN); Chengdu University of Traditional Chinese Medicine, Chengdu, Sichuan 611137 (CN)
(72) Inventor: PENG, Cheng, Chengdu Sichuan 611137 (CN); WAN, Feng, Chengdu Sichuan 610041 (CN); XIONG, Liang, Chengdu Sichuan 611137 (CN); LIN, Dasheng, Chengdu Sichuan 610041 (CN)
(74) Representative: Herzog, Fiesser & Partner Patentanwälte PartG mbB
(86) International application number: PCT/CN2013/080295
(87) International publication number: WO 2014/019485

(56) References cited:
- EP-A1- 2 826 473
- CN-A- 1 947 523
- CN-A- 101 481 370
- CN-A- 102 058 660
- CN-A- 102 058 660
- CN-A- 102 406 886
- Peng Fu Wan Feng Xiong Liang Peng Cheng Dai Min Chen Jianping ET AL: "In vitro and in vivo antibacterial activity of Pogostone", 中华医学?志& #xFF1A;英??, 1 January 2014 (2014-01-01), pages 4001-4005, XP055258635, China DOI: 10.3760/cma.j.issn.0366-6999.20140494 Retrieved from the Internet: URL:http://124.205.33.103:81/ch/reader/cre ate_pdf.aspx?file_no=20140494&flag=1&year_ id=2014&quarter_id=23 [retrieved on 2016-03-16]
- EDWARDS-JONES V ET AL: "The effect of essential oils on methicillin-resistant Staphylococcus aureus using a dressing model", BURNS, BUTTERWORTH HEINEMANN, GB, vol. 30, no. 8, 1 December 2004 (2004-12-01), pages 772-777, XP004650469, ISSN: 0305-4179, DOI: 10.1016/J.BURNS.2004.06.006
- KLEIN E ET AL: "ISOLATION STRUCTURE AND SYNTHESIS OF DHELWANGIN", TETRAHEDRON LETTERS, vol. 27, 1969, pages 2279-2280, XP009189127, ISSN: 0040-4039
- OSAWA K ET AL: "Studies of the antibacterial activity of plant extracts and their constituents against periodontopathic bacteria.", THE BULLETIN OF TOKYO DENTAL COLLEGE FEB 1990, vol. 31, no. 1, February 1990 (1990-02), pages 17-21, XP009189124, ISSN: 0040-8891
- NO. 1 SECTION OF GUANGDONG INSTITUTE OF ANALYSIS ET AL.: 'The separation of antifungal component-Pogostone from traditional Chinese medicine Pogostemon cablin and its structure determination' CHINESE SCIENCE BULLETIN vol. 22, no. 7, July 1977, pages 318 - 320, XP008176424
- 'COMPILING GROUP OF CHINESE HERBOLOGY OF CHINA PHARMACEUTICAL UNIVERSITY' CHINESE HERBOLOGY vol. 2, March 1980, pages 940 - 943, XP008176435
- KONG, GENGXING: 'The separation of patchoulenone and its application in medicament' CHINA JOURNAL OF HOSPITAL PHARMACY vol. 6, no. 1, January 1986, pages 32 - 33, XP008176371
- DU, YIMIN ET AL.: 'Chemical components of Pogostemon cablin and research development on its pharmacologic action' TRADITIONAL CHINESE DRUG RESEARCH & CLINICAL PHARMACOLOGY vol. 9, no. 4, December 1998, pages 238 - 240, XP008178070
- EDITORIAL COMMITTEE OF GUANGDONG ZHONGYAOZHI GUANGDONG ZHONGYAOZHI vol. 1, May 1994, pages 427 - 429, XP008176523
- CHEN, HAIMING ET AL.: 'Determination ofpogostone in pogostemon cabline (Blanco) Benth by HPLC' JOURNAL OF GUANGZHOU UNIVERSITY OF TRADITIONAL CHINESE MEDICINE vol. 28, no. 6, November 2011, pages 645 - 647, XP008178069
- YI, YUYANG ET AL.: 'Synthesis and antimicrobial evaluation of pogostone and its analogues' FITOTERAPIA vol. 84, January 2013, pages 135 - 139, XP028968937
- LI, YUCUI ET AL.: 'Anti-Candida albicans activity and pharmacokinetics of pogostone isolated from Pogostemonis Herba' PHVTOMEDICINE vol. 20, no. 1, December 2012, pages 77 - 83, XP055189763

## Description

### Field of the invention

The invention relates to new uses of pogotone (patchoulenone) and its derivatives, and belongs to the fields of pharmaceuticals, healthcare food, food, cosmetics, disinfectors or daily chemical articles, etc

### Background of the invention

Bacterial infectious disease is a disease resulted from bacterial infections. Among the common clinical pathogens, gram-negative bacteria such as *Escherichia, Enterobacter* and *Pseudomona,* are predominant, but there also are gram-positive bacteria such as *Staphylococcus* and *Enterococcus.*

Currently, drugs commonly used for treatment of bacterial infectious diseases mostly are antibiotics and broad-spectrum antibacterials, such as imipenem, second-choice cefoperazone-sulbactam, vancomycin, and second-choice nitrofurantoin. All of them have great adverse effects and their heavy uses can cause drug-resistant, leading to the occurrence of super bacteria. Meticillin-resistant *Staphylococcus aureus* is a super bacteria, and it is only sensitive to vancomycin. Since its first discovery, it has spread worldwide and become one of important pathogenic bacteria responsible for nosocomial infections.

There is an urgent need for searching for new drugs.

Pogostone (C₁₂H₁₆O₄) is one of active components isolated from the volatile oil of traditional Chinese medicine *Pogostemon cablin* (Blanco Benth). Its chemical name is 4-hydroxy-6-methyl-3-(4-methylpentanoyl)-2*H*-pyran-2-one, with an CAS registry number 23800-56-8, and chemical formula is as below:

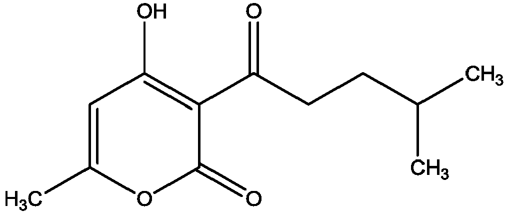

Pogostone is demonstrated to have antifungal properties, and it was disclosed to have certain inhibitory effect on fungus such as *Candida albicans, Cryptococcus neoformans*, *Rhizopus niger, Rhizoctonia leguminicola*, *Fusarium moniliforme*, *Curvularia lunata*, *Sclerotinia scleroterum*, *Pestalotia eugenae*, *Alternaria solani,* et al by Xiao-Lu Mo, et al in "(Study on the inhibitory activity of patchouli oil against plant disease fungus". Pogostone was reported to have good inhibitory effect on fungus by Geng-Xing Kong, et al in "Isolation of pogostone and its uses in medicament", Chin. J. Hosp. Pharm. 6(1), 1986.

Further publications relating to the inhibitory effects of Pogostone on microorganisms are CN 102 058 660 A, CN 1 947 523 A, CN 102 406 886 A, Osawa et al. (1990), Bull of the Tokyo Dental college (31).17; "Compiling group of Chinese herbology of China Pharmaceutical University" (1980) Chinese Herbology 2: 940; Kong et al. (1986), Chin J Hosp Pharmacy 6:32; Du et al. (1998), Trad Chin Drug Res & Clin Pharmacol 9:238; Editoraly Committe of Guangdong Zhonggyoazhi (1994), Guangdong Zhonggyoazhi 1:427; Chen et al. (2011), Journal of Guangzhou Univ of Traditional Chinese Med 28:645.

### Content of the invention

The present invention relates to non-therapeutic and therapeutic uses of pogostone as specified in the claims.

Technical protocols of the present disclosure provide new uses of pogostone and its derivatives.

The present disclosure provides the uses of pogostone and its derivatives in the preparation of antibacterial drugs, healthcare food, food, cosmetics, disinfectors or daily chemical articles.

In which, said drugs, healthcare food, food, cosmetics, disinfectors or daily chemical articles are those used for prevention, treatment or adjunctive therapy of bacterial infections.

In which, said bacteria are drug resistance bacteria.

In which, said bacteria are gram-positive or gram-negative bacteria.

Said gram-positive bacteria are those belonging to *Staphylococcus*, *Enterococcus*, *Streptococcus*, *Kurthia*, *Mycobacterium, Macrococcus*, *Listeria monocytogenes*, *Bacillus anthracis*, *Erysipelothrix*, *Renibacterium*, *Bacillus*, *Clostridium, Actinomyces, Nocardia, Corynebacterium,* or *Rhodococcus.*

Said gram-negative bacteria are those belonging to *Pseudomonas, Aeromonas, Enterobacter, Acinetobacter, Flavobacterium, Proteus, Escherichia, Shigella,* or *Klebsiella.*

Said *Staphylococcus* bacteria are *Staphylococcus haemolyticus, Staphylococcus xylosus*, *Staphylococcus saprophyticus*, *Staphylococcus auricularis*, *Staphylococcus aureus*, *Staphylococcus epidermidis*, or *Staphylococcus wameri*;
Said *Enterococcus* bacteria are *Enterococcus faecalis* or *Enterococcus faecium*;
Said *Macrococcus* bacterium is *Megasphaera elsdenii*;
Said *Bacillus* bacterium is *Bacillus gibsonii*;
Said *Corynebacterium* bacterium is *Corynebacterium xerosis*;
Said *Pseudomonas* bacterium is *Pseudomonas aeruginosa*;
Said *Aeromonas* bacterium is *Aeromonas caviae*;
Said *Enterobacter* bacterium is *Enterobacter cloacae*;
Said *Acinetobacter* bacterium is *Acinetobacter lwoffii*;
Said *Flavobacterium* bacterium is *Chryseobacterium indologenes*;
Said *Proteus* bacterium is *Proteus vulgaris*;
Said *Escherichia* bacterium is *Escherichia coli*;
Said *Shigella* bacterium is *Shigella flexneri*;
Said *Klebsiella* bacterium is *Klebsiella pneumoniae.*

In which, said drug-resistant bacteria are methicillin-resistant bacteria. Said drug-resistant bacteria are methicillin-resistant *Staphylococcus aureus.*

Said daily chemical articles are bathroom products or abluents. Said bathroom products are soap, toothpaste, body wash, hand sanitizer, laundry liquid, or dentilave; said abluents are liquefied detergent.

For the antibacterial drug, healthcare food, food, cosmetics, disinfector or daily chemical article according to the present disclosure, it is prepared using pogostone and its derivatives as active components, with the addition of adjuvants or auxiliary constituents acceptable in the field of pharmacy, healthcare food, food, cosmetics, disinfector or daily chemical articles.

Said preparations are liquid preparations, gas preparations, solid preparations, or semisolid preparations.

Pogostone and its derivatives according to the present invention are natural medicines, and have good inhibitory effects on pathogenic bacteria (such as *Staphylococcus,* etc) or conditional pathogenic bacteria. It can be used for treatment of bacterial infectious diseases and also be active against methicillin-resistant bacteria. Antibiotics may result in drug-resistance of pathogenic bacteria, but pogostone can overcome their deficits, and be considered as a potential pioneering antibacterial agent for clinical application.

Obviously, according to above contents of the present disclosure, as well as general technical knowledges and commonly-used means in the art, many other modifications, replacement or variations can be made.

The following examples describe the present invention in detail, but are not to be construed to be in any way limiting for the present invention. Any technologies that can be performed based on above contents are all within the scope of the present disclosure.

### Examples

Pogostone used in the present invention is commercially available, and its purity was determined to be above 98% by assaying.

### Example 1 The preparation of dentilave according to the present invention

Pogostone was mixed with propylene glycol, Tween-60, distilled water, glycerol, surface-active agents, ispropanol, and then uniformly dispersed. After filtration, the filtrate was packaged, to obtain dentilave.

### Example 2 The preparation of cosmetics according to the present invention

Pogostone was uniformly mixed with suitable amounts of lanolin, glycerin monostearate, vaseline, cera chinensis, stearic acid, liquid paraffin, benzoic acid, triethanolamine, propylene glycol, distilled water, Tween-60, sodium metabisulfite, essence, preservatives, cerebrin, fruit acid sodium, hexadecanol, and then stored under cooling, to obtain cosmetics.

### Example 3 The preparation of antiseptic solution according to the present invention

A suitable amount of pogostone was taken, and then uniformly mixed with Tween-60, carboxymethylcellulose sodium, polyethylene glycol 200, and distilled water, to obtain antiseptic solution.

### Example 4 The preparation of daily chemical articles-abluents according to the present invention

Pogostone was mixed with suitable amounts of carboxylic acid polymers, nonionic wetting agents, anionic surfactants, enzymes, enzyme stabilizers, foam control agents, Tween-80, and water, to prepare abluents.

### Example 5 The preparation of daily chemical articles-hand sanitizer according to the present invention

Pogostone was mixed with glycerol, sodium benzoate, sodium chloride, deionized water, essence, triethanolamine, glyceryl monostearate, hydrogenated lanolin, stearic acid, ethylparaben, mechelie essence, and then uniformly dispersed, followed by bulking, to obtain hand sanitizer.

### Example 6 Food according to the present invention

Pogostone was mixed with red babyberry and then suitable amounts of Tangerine Pericarp *pulveratus*, *Cinnamomi cortex* pulveratus, granulated sugar, *Flos caryophylli* pulveratus, licorice pulveratus, fennel pulveratus, table salt, and alumen were added, to prepare red babyberry preserves.

Once red babyberry was prepared well, a layer of 20 cm thick of red babyberry was firstly placed, then a layer of mixed table salt, alumen, and pogostone was placed, and sequentially they were pressed hard. Afterward, similarly, a layer of red babyberry was placed, followed by a layer of mixtures of table salt, alumen, and pogostone, and then they were pressed hard and soused, to obtain red babyberry base. Red babyberry base was taken and added with granulated sugar, perfume pulveratus, Tangerine Pericarp pulveratus, *Cinnamomi cortex* pulveratus, *Flos caryophylli* pulveratus, licorice pulveratus, fennel pulveratus, followed by immersion in water, drying in the sun, mixing, package, to obtain red babyberry preserves.

### Example 7 Healthcare food according to the present invention

Pogostone was taken and added with suitable amounts of carboxymethylcellulose sodium, that was uniformly mixed with suitable amounts of capillary wormwood herb, Villous Amonmum Fruit, medlar, Finger Citron Fruit, *Folium isatidis* fresh tea, chrysanthemum, *Semen cassiae,* honeysuckble flower bud, the rhizome of large-headed atractylodes, *Fructus corni,* root of herbaceous peony, mulberry leaf, Schisandra Chinensis, fennel, common yam rhizome, to prepare hepatoprotective tea, and all of the agents added to the mixture had already been withered using withering trough, de-enzymed with fixing machine, and rolled by rolled machine.

### Example 8 The preparation of drug granules according to the present invention

After addition of pogostone, suitable amounts of starch and dextrin were added, and then the mixture was granulated, to obtain granules.

Beneficial effects of the present invention was illustrated via following examples.

### Experimental example 1 Study on the in vitro antibacterial activity of pogostone

### 1 Materials and apparatus

### 1.1 Laboratory apparatus

Multipoint inoculation apparatus (Sakuma Manufacturing of Japan, SAKUMA MIT-P type), electric-heated thermostatic water bath (Beijing Tonghua Medical Apparatus and Instruments Factory, DSY-1-6 hole), Laboratory High Pressure Sterilizer (SANYO, MLS-3780 type), CO₂ INCUBATOR (SANYO, MOC-15A), Saving Energy Microbench (Chengdu Xinguang Feilante Purification Engineering Co. Ltd).

### 1.2 Experimental strains

*Staphylococcus aureus* ATCC 25923; methicillin-resistant *Staphylococcus aureus* ATCC 43300; *Escherichia coli ATCC* 25922.

All of above standard quality control strains were provided by Sichuan Industrial Institute of Antibiotics.

*Staphylococcus haemolyticus*, *Staphylococcus xylosus*, *Megasphaera elsdenii*, *Staphylococcus saprophyticus*, *Staphylococcus auricularis*, *Corynebacterium xerosis*, *Staphylococcus aureus*, *Aeromonas caviae*, *Enterobacter cloacae*, *Acinetobacter lwoffii*, *Chryseobacterium indologenes*, *Proteus vulgaris*, *Escherichia coli*, *Staphylococcus epidermidis*, *Staphylococcus wameri*, *Bacillus gibsonii*, *Enterococcus faecalis*, methicillin-resistant *Staphylococcus aureus*, *Enterococcus faecium*, *Shigella flexneri*, *Pseudomonas aeruginosa*, *Klebsiella pneumoniae*, et al.

All of above strains were mostly clinically isolated from the blood, phlegm, urine, etc. of patients who were treated at first rate hospitals in Chengdu district of Sichuan province and Beijing district from October 2010 to January 2012. Samples were identified via BioMeriruk VITEK-32 and VITEK-60 automatic microorganism assay analyzer (France) in above hospitals, and further confirmed using Biolog bacterial identification instrument (USA) as well as API 20E, 20NE, Staph series and general methods in laboratory before being used in the experiment.

### 1.3 Drugs

**Tested drug:** pogostone.
**Positive drugs:** Benzylpenicillin sodium for injection (North China Pharmaceutical Co. Ltd, batch number: Y1104101); Cefotaxime sodium for injection (Northeast Pharmaceuticals, batch number: 0910203).

### 1.4 Culture media, reagents and consumables

Mueller-Hinton agar (OXOID, batch number: 729683), nutrient agar culture medium (Hangzhou Microbial Reagent Co., Ltd., batch number: 20100831-02), sodium chloride (Tianjin Damao Chemical Reagent Factory, batch number: 20080328), MILLEX-GP Filter Unit (0.22 µm), disposable sterile petri dish (90 mm, purchased from Jiangsu Kangjian Biotechnology Co., Ltd.), Mcfarland Standard (bioMeriéux, Inc., batch number: 821772701), etc.

### 2 Experimental methods

Preparation of drug-containing plates: pogostone was dissolved in DMSO, and then diluted using DMSO double dilution method. 1 ml drug solutions at different gradient concentrations and 14 ml sterile MH culture media were respectively added to the disposable sterile petri dishes. Tested drugs were respectively diluted to different gradient concentrations, mixed in fully, and dried to reserve. As mentioned above, replacing drug with same amount of physiological saline was used to prepare positive control plates.

Preparation of bacterial liquid: Tested strains and drug-sensitive strains used for quality control were diluted with normal saline solutioninto the concentration of 10⁶ CFU/ml.

*In vivo* antibacterial activity assay: Using multipoint inoculation apparatus, the tested strain liquid was added to the drug-containing plates in different gradient concentrations and the positive control plates, respectively, and replacing the bacterial liquid with 5 µL normal saline solution was used as the negative control.

The strains were incubated at 37 □ for 18-24 h, and results were observed and recorded.

Result assessment: The minimum concentration of drug in plates of no bacterial growth was regarded as the minimum inhibitory concentration (MIC).

### 3. Experimental results

The results were shown in Tables 1-3.

**Table 1 Bacteriostasis results**

| Classification | Bacteria strains | MIC (µg/ml) | | |
|---|---|---|---|---|
| | | Pogostone | Penicillin | Cefotaxime sodium |
| Gram positive bacteria | *Staphylococcus haemolyticus* 5-4 | 400 | <0.098 | 0.195 |
| | *Staphylococcus xylosus* 5-5 | 200 | <0.098 | 0.391 |
| | *Megasphaera elsdenii* 5-6 | 200 | 0.781 | 6.25 |
| | *Staphylococcus saprophyticus* 5-7 | 200 | <0.098 | 0.391 |
| | *Staphylococcus xylosus* 5-8 | 200 | <0.098 | 0.391 |
| | *Staphylococcus auricularis* 5-9 | 400 | <0.098 | 3.125 |
| | *Corynebacterium xerosis* 5-10 | <0.098 | 1.563 | 1.563 |
| | *Staphylococcus aureus* ATCC 25923 | 400 | <0.098 | <0.098 |
| | *Staphylococcus aureus* 5-11 | 400 | <0.098 | 1.563 |
| Gram negative bacteria | *Pseudomonas aeruginosa* 5-4 | 100 | <0.098 | 25 |
| | *Aeromonas caviae* 5-5 | 1600 | 400 | <0.098 |
| | *Enterobacter cloacae* 5-4 | 400 | 0.781 | 25 |
| | *Enterobacter cloacae* 5-5 | 200 | 0.781 | 25 |
| | *Acinetobacter lwoffii* 5-4 | 200 | <0.098 | 6.25 |
| | *Acinetobacter lwoffii* 5-5 | 200 | <0.098 | 6.25 |
| | *Chryseobacterium indologenes* 5-4 | <0.098 | 50 | 25 |
| | *Chryseobacterium indologenes* 5-5 | 100 | 25 | 25 |
| | *Chryseobacterium indologenes* 5-6 | 50 | 50 | 25 |
| | *Chryseobacterium indologenes* 5-7 | 200 | 50 | 25 |
| | *Chryseobacterium indologenes* 5-8 | 200 | 25 | 25 |
| | *Proteus vulgaris* 5-4 | 400 | <0.098 | 0.391 |
| | *Escherichia coli* 5-4 | 1600 | 12.5 | <0.098 |
| | *Escherichia coli* 5-5 | 6.25 | 12.5 | <0.098 |
| | *Escherichia coli* 5-6 | 400 | <0.098 | 3.125 |
| | *Escherichia coli* 5-7 | 200 | <0.098 | 0.391 |
| | *Escherichia coli* ATCC 25922 | 400 | 0.098 | 1.563 |

**Table 2 Bacteriostasis results**

| Classification | Bacteria strains | MIC (µg/ml) |
|---|---|---|
| | | Pogostone |
| | *Staphylococcus epidermidis* 6-1 | 400 |
| | *Staphylococcus epidermidis 6-2* | 400 |
| | *Staphylococcus auricularis* 6-1 | 200 |
| | *Staphylococcus auricularis 6-2* | 400 |
| | *Staphylococcus auricularis* 6-3 | 200 |
| | *Staphylococcus saprophyticus* 6-1 | 800 |
| | *Staphylococcus haemolyticus* 6-1 | 400 |
| | *Staphylococcus wameri* 6-1 | 400 |
| | *Bacillus gibsonii* 6-1 | 200 |
| | Macrococcus 6-1 | 200 |
| Gram positive bacteria | Macrococcus 6-2 | 200 |
| | Macrococcus 6-3 | 200 |
| | Enterococcus faecalis6-1 | 200 |
| | *Staphylococcus xylosus* 6-1 | 400 |
| | *Staphylococcus xylosus* 6-2 | 400 |
| | *Staphylococcus aureus* 6-1 | 400 |
| | *Staphylococcus aureus* 6-2 | 200 |
| | *Staphylococcus aureus* 6-3 | 200 |
| | *Staphylococcus aureus* 6-4 | 400 |
| | *Staphylococcus aureus* 6-5 | 400 |
| | *Staphylococcus aureus* 6-6 | 200 |
| | *Staphylococcus aureus* 6-7 | 400 |
| | *Staphylococcus aureus* 6-8 | 400 |
| | methicillin-resistant *Staphylococcus aureus* 6-9 | 400 |
| | *Staphylococcus aureus* ATCC25923 | 400 |
| | methicillin-resistant *Staphylococcus aureus* ATCC43300 | 400 |
| | *Enterococcus faecalis* 6-1 | 400 |
| | *Enterococcus faecium* 6-2 | 200 |

**Table 3 Bacteriostasis results**

| Classification | Bacteria strains | MIC (µg/ml) |
|---|---|---|
| | | Pogostone |
| | *Acinetobacter lwoffii* 6-1 | 200 |
| | *Acinetobacter lwoffli* 6-2 | 200 |
| | *Escherichia coli* 6-1 | >800 |
| | *Escherichia coli* 6-2 | 400 |
| | *Escherichia coli* 6-3 | >800 |
| | *Escherichia coli* 6-4 | >800 |
| | *Escherichia coli* 6-5 | 400 |
| | *Escherichia coli* 6-6 | >800 |
| | *Escherichia coli* 6-7 | >800 |
| | *Escherichia coli* 6-8 | >800 |
| | *Escherichia coli* 6-9 | >800 |
| | *Escherichia coli* 6-10 | >800 |
| | *Escherichia coli* 6-11 | >800 |
| | *Escherichia coli* 6-12 | >800 |
| Gram negative bacteria | *Escherichia coli* 6-13 | 800 |
| | *Escherichia coli* 6-14 | 800 |
| | *Proteus vulgaris* 6-1 | 400 |
| | *Shigella flexneri* 6-1 | 400 |
| | *Chryseobacterium indologenes* 6-1 | 100 |
| | *Chryseobacterium indologenes 6-2* | 200 |
| | *Chryseobacterium indologenes* 6-3 | 100 |
| | *Chryseobacterium indologenes* 6-4 | 400 |
| | *Pseudomonas aeruginosa* 6-1 | >800 |
| | *Pseudomonas aeruginosa* 6-2 | >800 |
| | *Pseudomonas aeruginosa* 6-4 | 100 |
| | *Enterobacter cloacae* 6-1 | >800 |
| | *Enterobacter cloacae* 6-2 | >800 |
| | *Enterobacter cloacae* 6-3 | >800 |
| | *Enterobacter cloacae* 6-4 | 200 |
| | *Enterobacter cloacae* 6-5 | 100 |
| | *Klebsiella pneumoniae* 6-1 | 400 |
| | *Klebsiella pneumoniae* 6-2 | 200 |

According to Tables 1-3, pogostone showed good antimicrobial activity against test strains such as *Staphylococcus haemolyticus, Staphylococcus xylosus, Macrococcus*, *Staphylococcus saprophyticus*, *Staphylococcus auricularis, Corynebacterium xerosis*, *Staphylococcus aureus*, *Pseudomonas aeruginosa*, *Aeromonas caviae*, *Enterobacter cloacae*, *Acinetobacter lwoffii*, *Chryseobacterium indologenes*, *Proteus vulgaris*, *Escherichia coli, Staphylococcus epidermidis*, *Staphylococcus wameri*, *Bacillus gibsonii*, *Enterococcus faecalis*, methicillin-resistant *Staphylococcus aureus*, *Enterococcus faecium*, *Shigella flexneri*, *Pseudomonas aeruginosa*, *Klebsiella pneumoniae*, etc, with MIC values of 100-400 µg/ml. Its MIC values against *Escherichia coli* reached up to 6.25 µg/ml, while MIC values against *Corynebacterium xerosis* and *Chryseobacterium indologenes* are less than 0.098 µg/ml.

The above results indicated pogostone showed good antibacterial activity against both sensitive strains or drug-resistant strains of Gram-positive bacteria and Gram-negative bacteria, and especially showed better effect against *Escherichia coli, Corynebacterium xerosis* and *Chryseobacterium indologenes*.

### Experimental example 2 In vivo antibacterial activity of pogostone

### 1 Experimental materials

### 1.1 drugs

Tested drug, Pogostone;
Positive drug, Benzylpenicillin sodium injection, specifications: 8×10⁵ units, North China Pharmaceutical Co. Ltd, batch number: Y1104101; vancomycin hydrochloride injection, specifications: 5×10⁵ units, Xinchang Pharmaceutical Factory of Zhejiang Pharmaceutical Co. Ltd, batch number: 111204; Cefotaxime sodium injection, specifications: 1.0 g, Hebei Huamin Pharmaceutical Co., Ltd. of Northeast Pharmaceuticals, batch number: XF3120707.

Drug for making model: Mucin form Porcine Stomach (Type □), specification: 100 g, SIGMA-ALDRICH, batch number: 018K0079.

### 1.2 Strains

Meticillin-resistant *Staphylococcus aureus* ATCC 43300, *Escherichia coli* ATCC 25922, provided by Sichuan Industrial Institute of Antibiotics.

### 1.3 Animals

Male and female (1:1) KM mice (grade SPF) weighing (20±2) g were obtained from Sichuan Institute of Biological Products, with certification number SCXK (Chuan) 2008-08.

### 2 Experimental contents

### 2.1 Preliminary test

Tested strains' virulence assay: Meticillin-resistant *Staphylococcus aureus* and *Escherichia coli* were cultured in incubator at 37 □ to reach log phase of growth, and then diluted in sterile saline to different gradient concentrations, and mixed with equivalent 10% gastric mucin. The mixed solution of each strain was administrated to mice at 0.5 ml per 20 g by intraperitoneal injection, and for the control group, each mouse was injected with 5% gastric mucin at the same dose. Under a premise of no control mice death, the mortality rate of mice within 14 days was recorded, and the minimum bacterial volumes leading to death of whole mice were measured, i.e. the minimum lethal dose (MLD) of bacterial liquid.

Preliminary test of effective dose *in vivo*: Infection model mice respectively infected by Meticillin-resistant *Staphylococcus aureus* and *Escherichia coli* were randomly divided into a few of groups (four mice per group), and then the obtained bacterial solution of each strain was intraperitoneally administrated to mice of experimental groups once a day, after antibactial agents were given intraperitoneally for one day, and the challenge was not conducted until 0.5 h after administration on the second day. The mortality rate of mice within 14 days was observed in each group, to determine the dosage used in the formal experiment.

### 2.2 In vivo antibacterial activity

KM mice (grade SPF) weighing 18-22 g were randomly divided into nine groups, and each group included five male and five female mice. In particular, there were three antibiotic groups (benzylpenicillin sodium injection, vancomycin hydrochloride injection, and Cefotaxime sodium injection); three experimental groups (high, middle, and low doses); Blank control group; model group; and control group receiving gastric mucin. All of mice were administrated by intraperitoneal injection once a day, after antibactial agents were given intraperitoneally for two days, and the challenge was not conducted until 0.5 h after administration on the third day, via intraperitoneally injecting solutions of meticillin-resistant *Staphylococcus aureus* and *Escherichia coli* containing 5% gastric mucin. Amongst, mice in the model group were given the same volume of normal saline; mice in the *vehicle group were not given antibiotics and also not challenged; mice in the gastric mucin control group were only injected the same volume of 5% gastric mucin without bacteria.*

### 2.3 Statistical analysis

The mortality rate of mice within 14 days following challenge was recorded in each group, and its survival rate was accounted.

### 3 Experimental results

Meticillin-resistant *Staphylococcus aureus* and *Escherichia coli* were regarded as the representative strains for Gram positive and negative bacteria, respectively, and the obtained results were shown as follows.

### 3.1 Preliminary test

Results of preliminary test showed MLD values of mice against meticillin-resistant *Staphylococcus aureus* and *Escherichia coli* were 1.28×10⁷ CFU/g and 1.17×10⁴ CFU/g, respectively; high, middle, and low doses of pogostone used in *in vivo* antibacterial test in mice were determined as 100, 50, and 25 mg/kg, respectively.

### 3.2 In vivo antibacterial activity

**Table 4 In vivo protective activity of pogostone for mice infected by methicillin-resistant Staphylococcus aureus.**

| Groups | Number *of* mice (n) | Drug injected (mg/Kg) | methicillin-resistant | *Staphylococcus aureus* |
|---|---|---|---|---|
| | | | Survival number(n) | Survival rate(%) |
| Blank control group | 10 | - | 10 | 100 |
| Model group | 10 | - | 0 | 0 |
| Gastric mucin control group | 10 | 1250 | 10 | 100 |
| benzylpenicillin sodium inj ection | 10 | 400000 units(240g)/Kg | 9 | 90 |
| vancomycin hydrochloride inj ection | 10 | 400000 units(400g)/Kg | 10 | 100 |
| High dose pogostone group | 10 | 100 | 6 | 60 |
| Middle dose pogostone group | 10 | 50 | 5 | 50 |
| low dose pogostone group | 10 | 25 | 2 | 20 |

According to Table 4, intraperitoneal injection of pogostone showed good *in vivo* protective effect on infected mice by meticillin-resistant *Staphylococcus aureus,* and the protective capacity on mice in groups of high and middle doses achieved 60% and 50%, respectively. While the group of low dose also showed protective effect, with an inhibitory value of 20%.

**Table 5 In vivo protective activity of pogostone for mice infected by Escherichia coli.**

| Groups | Number of mice (n) | Drug injected (mg/Kg) | Escherichia coli | |
|---|---|---|---|---|
| | | | Survival number(n) | Survival rate(%) |
| Blank control group | 10 | - | 10 | 100 |
| Model group | 10 | - | 0 | 0 |
| Gastric mucin control group | 10 | 1250 | 10 | 100 |
| Cefotaxime sodium injection | 10 | 600 | 10 | 100 |
| High dose pogostone group | 10 | 100 | 9 | 90 |
| Middle dose pogostone group | 10 | 50 | 9 | 90 |
| low dose pogostone group | 10 | 25 | 6 | 60 |

According to Table 5, intraperitoneal injection of pogostone showed significant *in vivo* protective effect on infected mice by *Escherichia coli,* and the protective capacity on mice in groups of high and middle doses both achieved 90%, while the protective capacity on mice in the group of low dose was still 60%.

In summary, pogostone showed good antibacterial activity, and can be effectively used for treatment of bacterial infectious diseases. Meanwhile, pogostone also can effectively inhibit meticillin-resistant bacteria, and it showed an efficient protective capacity both *in vivo* and *in vitro*, providing a potential pioneering antibacterial agent for clinical application.

## Claims

1. Non-therapeutic use of Pogostone as an antibacterial disinfectant, wherein said bacteria are drug-resistant, wherein said drug-resistant bacteria are methicillin-resistant *Staphylococcus aureus.*

2. The non-therapeutic use of Pogostone according to claim 1, **characterized in that** said Pogostone is comprised in a disinfectant, laundry liquid, or liquefied detergent.

3. The non-therapeutic use of Pogostone of any one of claims 1 to 2, wherein said disinfectant, laundry liquid, or liquefied detergent further comprises adjuvants or auxiliary constituents acceptable in the field of disinfectants.

4. The non-therapeutic use of Pogostone of any one of claims 2 to 3, wherein said disinfectant, laundry liquid, or liquefied detergent is a liquid preparation, a gas preparation, a solid preparation, or a semisolid preparation.

5. Pogostone for use in prevention, treatment or adjunctive therapy of bacterial infections, wherein said bacteria are drug-resistant, wherein said drug-resistant bacteria are methicillin-resistant Staphylococcus aureus.

6. Pogostone for use according to claim 5, wherein said prevention, treatment or adjunctive therapy comprises preparing an antibacterial drug, healthcare food, food, cosmetics, or disinfector using pogostone as active component, with the addition of adjuvants or auxiliary constituents acceptable in the field of pharmacy, healthcare food, food, cosmetics, or disinfector.

## Patentansprüche

1. Nichttherapeutische Verwendung von Pogoston als antibakterielles Desinfektionsmittel, wobei die Bakterien arzneimittelresistent sind, wobei es sich bei den arzneimittelresistenten Bakterien um Methicillin-resistente *Staphylococcus aureus* handelt.

2. Nichttherapeutische Verwendung von Pogoston nach Anspruch 1, **dadurch gekennzeichnet, dass** das Pogoston in einem Desinfektionsmittel, einem Flüssigwaschmittel, oder einem verflüssigten Reinigungsmittel enthalten ist.

3. Nichttherapeutische Verwendung von Pogoston nach einem der Ansprüche 1 bis 2, wobei das Desinfektionsmittel, Flüssigwaschmittel, oder verflüssigte Reinigungsmittel ferner Adjuvantien oder Hilfsbestandteile umfasst, die im Gebiet der Desinfektionsmittel akzeptabel sind.

4. Nichttherapeutische Verwendung von Pogoston nach einem der Ansprüche 2 bis 3, wobei es sich bei dem Desinfektionsmittel, Flüssigwaschmittel, oder verflüssigten Reinigungsmittel um eine Flüssigzubereitung, eine Gaszubereitung, eine feste Zubereitung oder eine halbfeste Zubereitung handelt.

5. Pogoston zur Verwendung bei der Vorbeugung, Behandlung oder adjuvanten Therapie bakterieller Infektionen, wobei die Bakterien arzneimittelresistent sind, wobei es sich bei den arzneimittelresistenen Bakterien um Methicillin-resistente *Staphylococcus areus* handelt.

6. Pogoston zur Verwendung nach Anspruch 5, wobei die Zubereitung, Behandlung oder adjuvante Therapie die Herstellung eines antibakteriellen Arzneimittels, Gesundheitslebensmittels, Lebensmittels, von Kosmetika, oder eines Desinfektionsapparats, unter Verwendung von Pogoston als Wirkstoff umfasst, mit dem Zusatz von Adjuvantien oder Hilfsbestandteilen, die im Gebiet von Pharmazie, Gesundheitslebensmittel, Lebensmittel, Kosmetika oder Desinfektionsaparaten akzeptabel sind.

## Revendications

1. Utilisation non thérapeutique de pogostone comme désinfectant antibactérien, où lesdites bactéries sont résistantes aux antibiotiques, où lesdites bactéries résistantes aux antibiotiques sont constituées par *Staphylococcus aureus* résistant à la méthicilline.

2. Utilisation non thérapeutique de pogostone selon la revendication 1, **caractérisée en ce que** ladite pogostone est comprise dans un désinfectant, un liquide pour lessive, ou un détergent liquéfié.

3. Utilisation non thérapeutique de pogostone selon l'une quelconque des revendications 1 à 2, **caractérisée en ce que** ledit désinfectant, liquide pour lessive, ou détergent liquéfié comprend en outre des adjuvants ou des constituants auxiliaires acceptables dans le domaine des désinfectants.

4. Utilisation non thérapeutique de pogostone selon l'une quelconque des revendications 2 à 3, **caractérisée en ce que** ledit désinfectant, liquide pour lessive, ou détergent liquéfié est une préparation liquide, une préparation gazeuse, une préparation solide, ou une préparation semi-solide.

5. Pogostone destinée à une utilisation dans la prévention, le traitement ou la thérapie d'appoint d'infections bactériennes, **caractérisée en ce que** lesdites bactéries sont résistantes aux antibiotiques, où lesdites bactéries résistantes aux antibiotiques sont constituées par *Staphylococcus aureus* résistant à la méthicilline.

6. Pogostone destinée à une utilisation selon la revendication 5, **caractérisée en ce que** ladite prévention, ledit traitement ou ladite thérapie d'appoint comprend la préparation d'un médicament antibactérien, d'un aliment de soin de santé, d'un aliment, de produits cosmétiques, ou d'un désinfecteur, en utilisant de la pogostone comme composant actif, avec l'addition d'adjuvants ou de constituants auxiliaires acceptables dans le domaine de la pharmacie, des aliments de soin de santé, des aliments, des produits cosmétiques, ou des désinfecteurs.
